(19) Europäisches Patentamt
European Patent Office
Office européen des brevets

(11) EP 4 501 358 A1

(12) **EUROPEAN PATENT APPLICATION**
published in accordance with Art. 153(4) EPC

(43) Date of publication:
**05.02.2025 Bulletin 2025/06**

(21) Application number: **23781090.8**

(22) Date of filing: **31.03.2023**

(51) International Patent Classification (IPC):
***A61K 47/12*** *(2006.01)* ***A61K 9/08*** *(2006.01)*
***A61K 47/18*** *(2017.01)*

(52) Cooperative Patent Classification (CPC):
**A61K 9/08; A61K 47/12; A61K 47/18**

(86) International application number:
**PCT/JP2023/013657**

(87) International publication number:
**WO 2023/191082 (05.10.2023 Gazette 2023/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **31.03.2022 JP 2022060469**

(71) Applicant: **MEDRx Co., Ltd.**
**Higashikagawa-shi**
**Kagawa 769-2712 (JP)**

(72) Inventors:
- **NAKAE, Takashi**
  **Higashikagawa-shi, Kagawa 769-2712 (JP)**
- **MIWA, Yasushi**
  **Higashikagawa-shi, Kagawa 769-2712 (JP)**
- **HAMAMOTO, Hidetoshi**
  **Higashikagawa-shi, Kagawa 769-2712 (JP)**
- **TATSUMI, Noboru**
  **Higashikagawa-shi, Kagawa 769-2712 (JP)**
- **YAMAGATA, Yutaka**
  **Higashikagawa-shi, Kagawa 769-2712 (JP)**

(74) Representative: **Vossius & Partner**
**Patentanwälte Rechtsanwälte mbB**
**Siebertstrasse 3**
**81675 München (DE)**

# (54) FORMULATION HAVING IMPROVED ABSORBABILITY OF LOW-SOLUBLE AND/OR LOW-MEMBRANE-PERMEABLE DRUG

(57) The present invention provides a pharmaceutical composition comprising a drug with low solubility and/or low permeability and an ionic liquid, wherein the ionic liquid is an anion ingredient and a cation ingredient, the anion ingredient is oleic acid or isostearic acid, and the cation ingredient is selected from a cationic lipid as well as a transmucosal absorption enhancer or a gastrointestinal absorption enhancer comprising an ionic liquid, wherein the ionic liquid is an anion ingredient and a cation ingredient, the anion ingredient is oleic acid or isostearic acid, and the cation ingredient is selected from a cationic lipid.

[Fig. 8]

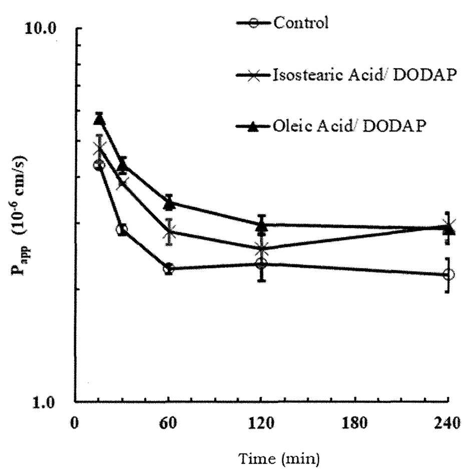


EP 4 501 358 A1

## Description

### TECHNICAL FIELD

The present invention relates to a preparation in which the absorbability of a drug with low solubility and/or low permeability is improved as well as a transmucosal absorption enhancer and a gastrointestinal absorption enhancer for improving the absorption of a drug with low solubility and/or low permeability to the transmucosal membranes and the gastrointestinal tract. Specifically, the present invention relates to a preparation comprising a drug with low solubility and/or low permeability and an ionic liquid in which the absorbability of the drug is remarkably improved as well as a transmucosal absorption enhancer and a gastrointestinal absorption enhancer for remarkably improving the absorption of a drug with low solubility and/or low permeability to the transmucosal membranes and the gastrointestinal tract, which comprise an ionic liquid.

### BACKGROUND ART

In the case of developing a drug as an oral preparation, the endpoints for drug efficacy are not achieved when the preparation does not produce sufficient oral absorbability, and thus the drug's development is delayed or stopped. Recently, in order to evaluate the risks, BCS (Biopharmaceutics Classification System) is used to predict factors limiting the oral absorbability of a drug from the property of an active pharmaceutical ingredient. In the BCS, compounds are classified into 4 classes based on the solubility and the permeability of a drug. The attempts to reveal the gastrointestinal absorption property of compounds in each class and use in the preparation development of the compounds have been made.

Specifically, in the BCS, compounds are classified into Class 1 (HS, HP) which is a group of compounds with high solubility (High Solubility; HS) and high permeability (High Permeability; HP), Class 2 (LS, HP) which is a group of compounds with low solubility (Low Solubility; LS) and high permeability (HP), Class 3 (HS, LP) which is a group of compounds with high solubility (HS) and low permeability (Low Permeability; LP), and Class 4 (LS, LP) which is a group of compounds with low solubility (LS) and low permeability (LP) based on a combination of the solubility of a compound in a buffer solution at pH 1.0 to 7.5 (250 mL) and the permeability of a compound produced by the permeability to membranes of cells such as Caco-2 cells derived from intestinal tract epithelial cells and MDCK cells or the permeability to membranes such as artificial membranes (Non-Patent Document 1). In particular, it is difficult to apply compounds with low solubility and/or low permeability, in which the absorbability to membranes such as transmucosal membranes and gastrointestinal tract is poor, as an oral preparation. Thus, the dosage forms used for the compounds are limited.

On the other hand, it has been desired in clinical environment to develop oral preparations that is less invasive and more convenient for patients. Currently, any methods useful for developing oral preparations comprising the compounds whose absorbability is improved have not been known.

Recently, as the attempts to improve the absorbability of compounds with low solubility and/or low permeability, the development of an agent for enhancing the absorbability of such compounds has been studied. Such agent is a compound that enhances the absorption of a drug, which acts directly on mucous membranes, changing their structure of mucous membranes. Many compounds, including surfactants, bile salts, bacterial toxins, chelating agents, and medium-chain fatty acids have been studied for their effectiveness in improving the absorbability of a molecule with low biological membrane permeability *in vitro* and *in vivo* tests. There are some compounds such as sodium caprate (C10) and salcaprozate sodium (SNAC) as absorption enhancers which are actually used in oral preparations. However, the compounds are mainly used in preparations comprising a protein such as GLP-1 and their effectiveness is not sufficient.

### PRIOR ART DOCUMENTS

### PATENT DOCUMENTS

Patent Document 1: JP 2020-73533
Patent Document 2: US 10278923

### NON-PATENT DOCUMENTS

Non-Patent Document 1: A theoretical basis for a biopharmaceutic drug classification: the correlation of in vitro drug product dissolution and in vivo bioavailability, Pharm Res. 1995 Mar; 12(3): 413-20.

## SUMMARY OF THE INVENTION

### PROBLEMS TO BE SOLVED BY THE INVENTION

The present inventors have developed many transdermal absorption preparations in which a compound such as low molecular compound, medium molecular nucleic acid and low molecular peptide is effectively absorbed transdermally using an ionic liquid (IL) prepared from an organic anion and an organic cation which is a salt in liquid form at ordinary temperature until now and have found that optimal ionic liquids for drugs with different properties can be designed. However, it is thought that since the mechanisms for absorbing drugs greatly differ between transdermal absorption and gastrointestinal absorption or intestinal absorption (transmucosal absorption), it is not expected to improve the absorbability of a drug using the ionic liquids used in transdermal absorption preparations.

An object of the present invention is to design an ionic liquid for enhancing the transmucosal absorbability and the gastrointestinal absorbability of a drug with low solubility and/or low permeability which are usually extremely difficult to be used for oral administration and to provide an oral preparation comprising such drug and ionic liquid in which the absorbability of the drug is remarkably improved as well as a transmucosal absorption enhancer and a gastrointestinal absorption enhancer for a drug with low solubility and/or low permeability whose transmucosal absorbability and gastrointestinal absorbability are poor.

### SOLUTIONS TO THE PROBLEMS

The present inventors have studied to find new ionic liquids for producing the effects of enhancing the intestinal absorption and the gastrointestinal absorption of drugs with low solubility and/or low permeability. In the study of ionic liquids, PROTAC (for example, BI-3663, VZ185) was used as model compounds of drugs with low solubility and/or low permeability.

The present inventors prepared various types of ionic liquids which may dissolve a drug with low solubility and/or low permeability and evaluated the permeability of such drug in a preparation comprising the prepared ionic liquid and the drug by any membrane permeation assay such as Parallel Artificial Membrane Permeation Assay (PAMPA) and the membrane permeation assay using Caco2 cells which are cell lines derived from human colon cancer and cell models that reproduce intestinal function in small intestine. The present inventors have found that the transmucosal absorbability and the gastrointestinal absorbability of a drug with low solubility and/or low permeability can be improved using specific ionic liquids. Based on the findings, the present invention has been completed.

That is, the present invention provides the following embodiments.

[Item 1] A pharmaceutical composition comprising a drug with low solubility and/or low permeability and an ionic liquid, wherein the ionic liquid comprises an anion ingredient and a cation ingredient, the anion ingredient is oleic acid or isostearic acid, and the cation ingredient is selected from a cationic lipid.

[Item 2] The pharmaceutical composition according to the above item 1, wherein the cationic lipid is DODAP, DODMA or DOTMA.

[Item 3] The pharmaceutical composition according to the above item 1 or 2, wherein the anion ingredient is oleic acid and the cation ingredient is DODAP or DODMA.

[Item 4] The pharmaceutical composition according to any of the items 1 to 3, wherein the drug with low solubility and/or low permeability is PROTAC.

[Item 5] An oral preparation comprising the pharmaceutical composition according to any of the above items 1 to 4.

[Item 6] A transmucosal absorption enhancer comprising an ionic liquid, wherein the ionic liquid comprises an anion ingredient and a cation ingredient, the anion ingredient is oleic acid or isostearic acid, and the cation ingredient is selected from a cationic lipid, provided that a drug is not comprised.

[Item 7] The transmucosal absorption enhancer according to the above item 6, wherein the cationic lipid is DODAP, DODMA or DOTMA.

[Item 8] The transmucosal absorption enhancer according to the above item 6 or 7, wherein the anion ingredient is oleic acid and the cation ingredient is DODAP or DODMA.

[Item 9] A preparation comprising a drug with low solubility and/or low permeability and the transmucosal absorption enhancer according to any of the above items 6 to 8.

[Item 10] The preparation according to the above item 9, wherein the drug with low solubility and/or low permeability is PROTAC.

[Item 11] A gastrointestinal absorption enhancer comprising an ionic liquid, wherein the ionic liquid comprises an anion ingredient and a cation ingredient, the anion ingredient is oleic acid or isostearic acid, and the cation ingredient is selected from a cationic lipid, provided that a drug is not comprised.

[Item 12] The gastrointestinal absorption enhancer according to the above item 11, wherein the cationic lipid is

DODAP, DODMA or DOTMA.

[Item 13] The gastrointestinal absorption enhancer according to the above item 11 or 12, wherein the anion ingredient is oleic acid and the cation ingredient is DODAP or DODMA.

[Item 14] An oral preparation comprising a drug with low solubility and/or low permeability and the gastrointestinal absorption enhancer according to any of the above items 11 to 13.

[Item 15] The oral preparation according to the above item 14, wherein the drug with low solubility and/or low permeability is PROTAC.

## EFFECTS OF THE INVENTION

According to the present invention, the absorbability of even a drug with low solubility and/or low permeability, which is difficult to be applied as an oral preparation, are remarkably improved, and thus the drug can be prepared as an oral preparation. In addition, a transmucosal absorption enhancer or a gastrointestinal absorption enhancer comprising the ionic liquid of the present invention can enhance the transmucosal or gastrointestinal absorption of a drug with low solubility and/or low permeability.

## BRIEF DESCRIPTION OF THE DRAWINGS

[Fig. 1] Fig. 1 shows the absorption-enhancing effects of each type of ionic liquid on VZ185 by the PAMPA. PBS was used as the control.

[Fig. 2] Fig. 2 shows the absorption-enhancing effects of each type of ionic liquid on BI-3663 by the PAMPA. PBS was used as the control.

[Fig. 3] Fig. 3 shows the absorption-enhancing effects of each type of ionic liquid on bifonazole by the PAMPA. PBS was used as the control.

[Fig. 4] Fig. 4 shows the absorption-enhancing effects (permeation coefficients) of each type of ionic liquid on VZ185 by the Caco-2 cell membrane permeation assay. Transport Buffer (A) was used as the control.

[Fig. 5] Fig. 5 shows the absorption-enhancing effects (permeation coefficients) of each type of ionic liquid on BI-3663 by the Caco-2 cell membrane permeation assay. Transport Buffer (A) was used as the control.

[Fig. 6] Fig. 6 shows the absorption-enhancing effect (permeation coefficient) of the ionic liquid (Oleic acid-DODMA) on bifonazole by the Caco-2 cell membrane permeation assay. Transport Buffer (A) was used as the control.

[Fig. 7] Fig. 7 shows the absorption enhancement effect (permeation coefficient) of the ionic liquid (Isostearic acid-DODMA) on ritonavir by the Caco-2cell membrane permeation assay. Transport Buffer (A) was used as the control.

[Fig. 8] Fig. 8 shows the absorption enhancement effects (permeation coefficients) of each type of ionic liquid on furosemide by the Caco-2 cell membrane permeation assay. Transport Buffer (A) was used as the control.

## DETAILED DESCRIPTION OF THE PREFERRED EMBODIMENTS

The present invention relates to a preparation comprising a drug with low solubility and/or low permeability and an ionic liquid in which the absorbability of the drug with low solubility and/or low permeability is remarkably improved as well as a transmucosal absorption enhancer and a gastrointestinal absorption enhancer for remarkably improving the transmucosal absorbability and the gastrointestinal absorbability of a drug with low solubility and/or low permeability, which comprise an ionic liquid.

In the present invention, the "drug with low solubility" is a drug whose solubility is less than or equal to the maximum amount of a drug contained in one unit of preparation in a buffer solution at pH 1.0 to 7.5 (250 mL) and includes, for example, a drug corresponding to Low Solubility (LS) defined in BCS. For example, the drug also includes a water-insoluble drug with a solubility of 100 μg or less in 1 mL of water, a drug that is difficult to use as injectable solution due to low solubility, and a drug that has low or no solubility in buffer solution and intestinal fluid or in the small intestine. The "drug with low solubility" may have the property as "drug with low permeability" because the low solubility of the drug reduces the absorption from transmucosal membranes and gastrointestinal tract. In the present invention, the "drug with low solubility" may have high permeability as long as it is a drug with low transmucosal and gastrointestinal absorbability

In the present invention, the "drug with low permeability" is a drug with low biomembrane permeability. Examples of the drug include a drug that is difficult to be absorbed through the gastrointestinal tract due to low permeability to the mucus layer presented on the gastrointestinal mucous membranes, a drug that is difficult to be absorbed due to the interaction with a substance in the mucus layer, or a drug that is difficult to be absorbed through the gastrointestinal tract due to low permeability in the gastrointestinal mucosa, a drug that is difficult to be absorbed orally due to the interaction with the gastrointestinal mucus layer and the mucous membranes, or a drug with low absorbability due to the formulation of

insoluble complexes with bile acid. Basically, the drug with low permeability refers to a drug that is difficult to be delivered by oral administration. In the present invention, the "drug with low permeability" may have high solubility as long as it is a drug with low gastrointestinal absorbability.

Also, examples thereof include a drug with low bioavailability, specifically a drug with an extent of bioavailability of 50% or less. The extent of bioavailability can be calculated according to the following formula.

Extent of Bioavailability (%) = 100 x (Amount of drug delivered in blood by oral administration)/(Amount of drug delivered in blood by intravenous administration)

Wherein the "amount of drug delivered in blood" is calculated as the area bounded by the blood concentration and the horizontal axis (time axis) (Area under drug blood concentration-time curve: AUC).

In addition, examples thereof include compounds that are determined to have low permeability by Single-layer membrane assay of cultured cells (such as Caco-2, MDCK, HT-29, LLC-PK1), Immobilized Artificial Membrane Chromatography assay, assay using distribution coefficients, Ribosomal Membrane assay, PAMPA (Parallel Artificial Membrane Permeation Assay).

In the present invention, the "drug with low solubility and/or low permeability" means a drug having both properties of the above "drug with low solubility" and "drug with low permeability". Examples thereof include a compound classified as Class 4 in BCS (for example, PROTAC). Also, there are the "drug with low solubility" that is low solubility in the small intestine and thus is difficult to be absorbed through the transmucosal membranes and gastrointestinal tract as well as the "drug with low permeability" that have high solubility but have low gastrointestinal absorbability. That is, the "drug with low solubility" or the "drug with low permeability" is included within the scope of the "drug with low solubility and/or low permeability". Examples thereof include BI-3663, VZ185, ACBI1, bifonazole, hydrochlorothiazide, furosemide, acetazolamide, sulfamethoxazole, chlorothiazide, enalaprilat, mebendazole, bifonazole, methotrexate, amphotericin B, ritonavir, but are not limited thereto.

In the present invention, the degree of absorbability of the drug with low solubility and/or low permeability is not particularly limited. A drug that produces no sufficient pharmacological effect when administered orally due to low transmucosal or gastrointestinal absorbability can produce the expected pharmacological effect by the increase in the oral absorbability of the drug. In addition, a drug with low transmucosal or gastrointestinal absorbability that produces sufficient pharmacological effect when administered orally can reduce the amount of the drug administered by further increase in the oral absorbability of the drug, and thus can achieve the reduction of side effects.

In the present invention, the "ionic liquid" is a salt prepared from an anion ingredient and a cation ingredient, which is in a viscous liquid from at ordinary temperature and is a melting point of 100°C or less, or means "hydrated ionic liquid". The "hydrated ionic liquid" means an ionic liquid composed of the ionic liquid and small amounts of water molecules. The ionic liquid may be prepared by mixing an anion ingredient and a cation ingredient in equimolar amounts or excess amounts at room temperature or with heating. The excess amounts of the anion ingredient and cation ingredient are preferably within 10-time molar amounts.

In the present invention, the "anion ingredient" is an organic anion. Examples thereof include an organic acid such as lactic acid, citric acid, tartaric acid, succinic acid, malic acid, benzoic acid, levulinic acid, aspartic acid, glutamic acid, octanoic acid, decanoic acid, lauric acid, myristic acid, stearic acid, behenic acid, isostearic acid, oleic acid, arachidonic acid, docosahexaenoic acid, eicosapentaenoic acid, linoleic acid, linolenic acid, adipic acid, and sebacic acid. In the present invention, the anion ingredient is preferably a long-chain fatty acid such as oleic acid and isostearic acid.

In the present invention, the "cation ingredient" is an organic cation. Examples thereof include a cationic lipid, an organic amine such as diethanolamine, triethanolamine, arginine, lysine, meglumine, trometamol, an organic quaternary ammonium cation, a basic amino acid, and an amino sugar. In the present invention, the cation ingredient is preferably a cationic lipid.

The cationic lipid broadly comprises hydrophobic and hydrophilic moieties. The hydrophobic moiety is a hydrophobic group such as fatty acid residue and sterol residue, and the hydrophilic moiety is an amino group or a cationic group such as ammonium group. In particular, many cationic lipids having the structure which has two hydrophobic groups in the hydrophobic moiety and one amino group or a cationic group such as ammonium group in the hydrophilic moiety (double chain cationic lipids) have been known.

In the present invention, examples of the cationic lipid include DODAP (1,2-dioleoyl-3-dimethylaminopropane), DODMA (1,2-dioleoyl-3-dimethylaminopropane), DOTMA (1,2-di-O-octadecenyl-3-trimethylammonium propane), DLinDAP (1,2-dilinoleoyl-3-dimethylaminopropane) and a salt thereof.

The ionic liquid of the present invention is prepared from a combination of the above anion ingredient and the above cation ingredient and may be an ionic liquid prepared by mixing two or more ionic liquids (including a mixed ionic liquid in which only the anion ingredient or the cation ingredient is different). Also, the ionic liquid may comprise water.

In the present invention, the "transmucosal absorption enhancer" means an agent for enhancing the transmucosal absorbability of a drug with low absorbability from mucous membranes other than gastrointestinal tract such as oral mucosa, rectum, vagina, nasal mucosa and lung (for example, a drug with low solubility and/or low permeability).

In the present invention, the "gastrointestinal absorption enhancer" means an agent for enhancing a drug with low gastrointestinal absorbability which is not effective to be delivered by oral administration (for example, a drug with low solubility and/or low permeability).

The preparation of the present invention may comprise a pharmaceutically acceptable carrier. Examples of the carrier include excipient, filming agent, binding agent, bulking agent, disintegrant, surfactant, lubricant, diluent, dispersant, buffer, osmotic regulator, pH adjuster, emulsifier, preservative, stabilizer, antioxidant, coloring agent, UV absorber, moisturizer, thickening agent, activity enhancer, anti-inflammatory agent, fungicide, flavoring agent, odor improving agent.

The carrier may be used alone and two or more carriers may be combined in appropriate amounts.

In addition, the preparation of the present invention may be formulated into the form of capsule comprising a water-soluble encapsulating agent. The water-soluble encapsulating agent is a substance for forming the film of a capsule. The water-soluble encapsulating agent seals content solution to produce the quality retention ability such as the antioxidation of the content solution, and it can be formulated as a capsule.

When the preparation of the present invention is formulated into a capsule, it may be formulated by a water-soluble macromolecule such as hydroxypropyl cellulose, hydroxypropyl methylcellulose, and polyvinylpyrrolidone.

EXAMPLES

Hereinafter, the present invention is specifically illustrated in Examples. The present invention, however, is not intended to be limited thereto by any means.

The physical properties and structures of BI-3663 and VZ185, which are drugs with low solubility and/or low permeability used in the working examples below, are described in the following table, but the drugs of the present invention are not limited to the compounds.

[Table 1]

| | Molecular weight | Solubility | Log P (Calculated value) | Target protein |
|---|---|---|---|---|
| BI-3663 | 918 | < 1 μg/ml | 3.6 | PTK2 |
| VZ185 | 995 | 85 μg/ml | 5.0 | BRD7/BRD9 |
| | Chemical structure | | | |
| BI-3663 | 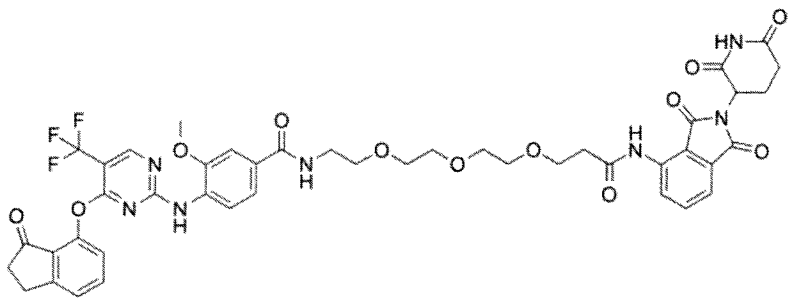 | | | |
| VZ185 | 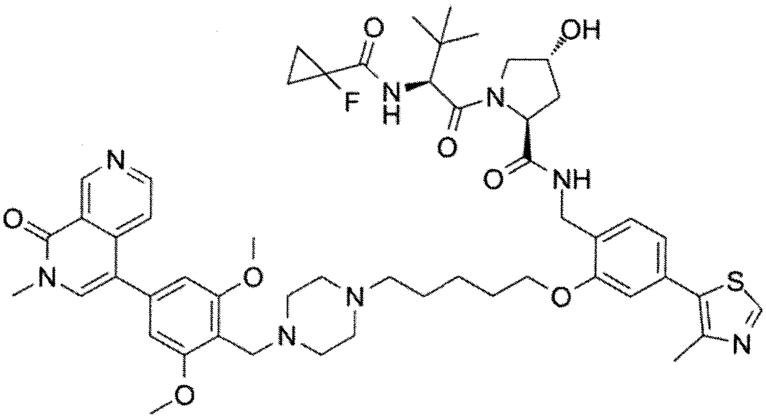 | | | |

Also, the drugs used in the working examples below, bifonazole, ritonavir and furosemide were purchased from Tokyo Chemical Industry Co., Ltd.

Example 1: Evaluation of permeability of drug by Caco-2 cell membrane permeation assay (1)

In order to study an ionic liquid for enhancing the intestinal absorption effect of a drug with low solubility and/or low permeability, various types of ionic liquids were prepared and the permeability of a drug with low solubility and/or low permeability in compositions comprising the prepared ionic liquid and the drug was evaluated. Specifically, the permeability of a drug was evaluated by the Caco-2 cell membrane permeation assay according to the following procedures for compositions comprising VZ185, which is PROTAC, used as a model drug with low solubility and/or low permeability and each ionic liquid. As controls, EDTA, SNAC used as absorption enhancer, sodium caprylate, sodium oleate, CAGE which has been reported that the mucosal absorption of insulin is enhanced, and lactic acid were used instead of the ionic liquid.

(1) Preparation of Transport Buffer

Sodium hydroxide (0.4 g) was dissolved in water (100 mL) to prepare 0.1N NaOH solution. MES (2-morpholinoethanesulfonic acid, 1.493 g) and D-glucose (2.453 g) were dissolved in HBSS (+) (free of phenol red) (600 mL), the solution was adjusted to pH 6.5 with 0.1N NaOH solution, and then HBSS (+) (free of phenol red) was further added and adjusted to 700 mL. The solution was sterilized by filtration through 0.22 μm membrane filter to prepare Transport Buffer (A).

HEPES (1.192 g), D-glucose (1.752 g) and BSA (1.25 g) were then dissolved in HBSS (+) (free of phenol red) (400 mL), the solution was adjusted to pH 7.4 with 0.1N NaOH solution, and then HBSS (+) (free of phenol red) was further added and adjusted to 500 mL. The solution was sterilized by filtration through 0.22 μm membrane filter to prepare Transport Buffer (B).

(2) Preparation of sample solution

VZ185 (1.5 mg) was mixed with 1% TWEEN 80 (1.5 mL) to 1 mg/mL of VZ185 solution. Each organic acid (0.05 mmol) which is an anion ingredient and each cationic lipid (0.05 mmol) which is a cation ingredient were mixed with Transport Buffer (A) (4.98 mL) to prepare an ionic liquid composition comprising 10 mM each of the ingredients. In addition, each of EDTA, SNAC, sodium caprylate, sodium oleate, CAGE (Choline and Geranate), and lactic acid in an amount of 0.05 mmol was mixed with Transport Buffer (A) (4.98 mL) to prepare 10 mM of each control composition as controls.

1 mg/mL of VZ185 solution (25 μL) and the ionic liquid composition or control composition (475 μL) were mixed to prepare 50 μg/mL of a drug-ionic liquid composition or a drug-control composition.

Caco-2 cells were then cultured on a 24 well Transwell for 3 weeks, 50 μg/mL of the drug-ionic liquid composition (100 μL) was added to the Apical side of each well and Transport Buffer (B) (600 μL) was added to the Basolateral side of each well, and then the mixture was incubated in 5% $CO_2$ incubator at 37°C. After 15, 30, 60, 120 and 240 minutes of the incubation, 200 μL of sample was collected from the Basolateral side of each well and the collected sample was used as sample solution. After collecting the sample, Transport Buffer (B) (200 μL) was added into each well, and then the mixture was incubated in 5% $CO_2$ incubator at 37°C again.

Also, control solution was prepared according to the same procedure as above, except that a drug-control composition was used instead of the drug-ionic liquid composition.

(3) Evaluation of cell membrane integrity

The membrane integrity of Caco-2 cells was evaluated by Trans-Epithelial Electrical Resistance (TEER) and Lucifer Yellow.

(3-1) Trans-Epithelial Electrical Resistance (TEER) was measured before and after the completion of the following Caco-2 membrane permeability evaluation. Specifically, Transport Buffer (A) (100 μL) was added to the Apical side of each well and Transport Buffer (B) (600 μL) was added to the Basolateral side of each well, and then the TEER value of each well was measured by Millicell ERS-2 (Merck Millipore Corporation, Darmstadt, Germany).

(3-2) The permeability of Lucifer Yellow was measured after the completion of the following Caco-2 membrane permeability evaluation. Specifically, Lucifer Yellow CH Dipotassium Salt (5.2 mg) was dissolved in Transport Buffer (A) (100 mL) to prepare 100 μM of Lucifer Yellow solution, 100 μM of the Lucifer Yellow solution (100 μL) was added to the Apical side of each well and Transport Buffer (B) (600 μL) was added to the Basolateral side of each well, and then the mixture was incubated in a 5% CO incubator at 37°C. After 60 minutes of the incubation, 200 μL of sample was collected from the Basolateral side of each well and the collected sample was used as sample for measuring fluorescence intensity. The sample for measuring fluorescence intensity (100 μL) and acetonitrile (400 μL) were mixed, the mixture was centrifuged at

4°C, 3,000 x g for 5 minutes, and then the concentration of Lucifer Yellow in the supernatant was measured by HPLC-FL under the condition in the table below.

[Table 2]

| | |
|---|---|
| HPLC system | LC-2010C HT (Shimadzu Corporation) |
| Fluorescence detector | RF-10A XL (Shimadzu Corporation) |
| Injection amount | 10 μL |
| Flow rate | 1 mL/min |
| Excitation wavelength (Ex) | 428 nm |
| Fluorescence wavelength (Em) | 538 nm |
| Analysis time | 1.5 mins |
| Eluent | $H_2O$/Acetonitrile = 1:1 |

(4) Evaluation of permeability

Donepezil hydrochloride (10 mg) was dissolved in methanol (100 mL) to prepare 100 μg/mL of donepezil hydrochloride solution. 100 μg/mL of the donepezil hydrochloride solution (1 mL) was accurately taken, methanol was added thereto and adjusted to exactly 100 mL, and the solution was used as IS standard undiluted solution. The IS standard undiluted solution (2 mL) was accurately taken, acetonitrile was added thereto and adjusted to exactly 200 mL, and the solution was used as IS-acetonitrile solution (10 ng/mL donepezil/acetonitrile solution).

The sample solution or control solution (100 μL) and IS-acetonitrile solution (400 μL) were mixed, the mixture was centrifuged at 4°C, 3,000 x g for 5 minutes, and then the concentration of drug in the supernatant was measured by LC-MS/MS under the condition in the table below.

[Table 3]

| | |
|---|---|
| HPLC system | LC-2040C (Shimadzu Corporation) |
| Mass spectrometer | LCMS8050 (Shimadzu Corporation) |
| Column | ACQUITYUPLC CSH C18 (2.1 x 50 mm I.D., 1.7 μm) |
| Column temperature | 40°C |
| Injection amount | 5 μL |
| Flow rate | 0.5 mL/min |
| Analysis time | 12 minutes |
| Eluent | A solution: 0.1% aqueous formic acid solution<br>B solution: 0.1% formic acid/acetonitrile |

[Table 4]

| Time program | | |
|---|---|---|
| Time (min) | A solution (%) | B solution (%) |
| 0 | 97 | 3 |
| 1 | 97 | 3 |
| 3.5 | 70 | 30 |
| 3.6 | 10 | 90 |
| 6.5 | 10 | 90 |
| 6.6 | 97 | 3 |

[Table 5]

| Setting condition of Multiple Reaction Monitoring (MRM) | |
|---|---|
| Precursor ion | Product ion |
| 996.15 | 309.15 |
| 919.00 | 442.95 |

The results are shown in Table 6. The TEER value > 1000 Ω and the Lucifer Yellow value < 1% were defined as standard values and the values were used as indexes for evaluating the membrane integrity. For the permeability, the membrane permeation coefficient ($P_{app}$) (x$10^6$cm/s) was calculated from the measured drug concentration according to the following Calculation Formula.

(Calculation Formula)

$$\text{Membrane Permeation Coefficient } (P_{app}) = (dQ/dt) \text{ x } \{1/(A \text{ x } C_0)\}$$

dQ/dt: Cumulative permeation amount on the Basolateral side
A: Membrane area of Transwell (0.33$cm^2$)
$C_0$ : Initial concentration of drug on the Apical side

[Table 6]

| | | | $P_{app}$ ($\times10^{-6}$ cm/s) | |
|---|---|---|---|---|
| Control | TEER | Lucifer Yellow (%) | at 0.5h | at 4h |
| EDTA | 225 | 2.2% | 0.8 | 1.2 |
| SNAC | 210 | 4.3% | 0.7 | 0.7 |
| Sodium caprylate | 620 | 2.9% | 1.0 | 4.4 |
| Sodium oleate | 100 | 0.1% | 0.0 | 0.0 |
| CAGE | 375 | 2.0% | 1.5 | 0.3 |
| Lactic acid | 550 | 0.5% | 2.5 | 0.7 |
| | | | $P_{app}$ ($\times10^{-6}$ cm/s) | |
| Ionic Liquid | TEER | Lucifer Yellow (%) | at 0.5h | at 4h |
| Decanoic acid-DODAP (5 mM)[1] | 1900 | 0.1% | 6.6 | 1.3 |
| Isostearic acid-DODAP (5 mM)[1] | 2500 | 0.1% | 11.2 | 2.2 |
| Oleic acid-DODAP (5 mM)[1] | 5465 | 0.0% | 8.8 | 1.1 |
| Oleic acid-Arginine | 3560 | 0.1% | 2.7 | 0.3 |
| oleic acid-Arginine methyl ester | 670 | 0.4% | 9.8 | 1.4 |
| Oleic acid-Histidine methyl ester | 4550 | 0.0% | 2.3 | 0.9 |
| Oleic acid-Glycine ethyl ester | 1660 | 0.1% | 3.8 | 0.5 |
| Oleic acid-Methylglycine ethyl ester | 3400 | 0.1% | 2.8 | 0.5 |
| Oleic acid-Serine methyl ester | 6660 | 0.0% | 2.8 | 0.4 |
| Oleic acid-Meglumine (1:2)[2] | 3515 | 0.1% | 3.6 | 0.6 |
| Oleic acid-Trometamol | 4750 | 0.1% | 2.4 | 0.4 |
| Lactic acid-Choline | 2150 | 0.3% | : 2.4 | 0.9 |

(continued)

| Ionic Liquid | TEER | Lucifer Yellow (%) | $P_{app}$ ($\times 10^{-6}$ cm/s) | |
|---|---|---|---|---|
| | | | at 0.5h | at 4h |
| Lactic acid-Triethanolamine | 1360 | 0.2% | 4.6 | 1.1 |
| Lactic acid-N,N-Dimethylglycine ethyl ester | 400 | 0.9% | 4.7 | 1.7 |
| Lactic acid-Proline methyl ester | 400 | 0.6% | 2.1 | 2.0 |
| Standard value | > 1000Ω | < 1% | | |
| [1]: Ionic liquid composition contained 5mM. Each ingredient is a half of the amount.<br>[2]: Ionic liquid composition comprising 10mM of oleic acid and 20mM of meglumine | | | | |

The results showed that the ionic liquid groups tended to have higher $P_{app}$ values although the ionic liquid groups had higher TEER values and lower Luciffer Yellow permeability as compared to the control groups, in particular, the ionic liquids: Decanoic acid-DODAP, Oleic acid-DODAP and Isostearic acid-DODAP had remarkably higher drug permeation coefficient. Hence, it was suggested that the ionic liquids in which a long-chain fatty acid and a cationic lipid are combined produced remarkable permeation-enhancing effects.

Example 2: Evaluation of permeability of drug by Caco-2 cell membrane permeation assay (2)

The results of Example 1 suggested that the ionic liquids in which a long-chain fatty acid and a cationic lipid are combined produced remarkable permeation-enhancing effects. Thus, the Caco-2 permeability and the cell membrane integrity were evaluated for various types of ionic liquids comprising DHA (C22), EPA (C20), arachidonic acid (C20), isostearic acid (C18), linoleic acid (C18) or linolenic acid (C18) as an anion ingredient and DODAP, DODMA or DOTMA as a cation ingredient. Specifically, the permeability of a drug was evaluated according to the same procedure as that of Example 1. As the control, Transport Buffer (A) prepared in Example 1 was used instead of the ionic liquid.

The results are shown in Table 7 below. The TEER value > 1000 Ω and the Lucifer Yellow value < 1% are desired values for the membrane integrity. Hence, the values were defined as standard values and it was determined to be good when exceeding the standard values. For the permeability, the membrane permeation coefficient ($P_{app}$) ($\times 10^6$cm/s) was calculated from the measured drug concentration according to the Calculation Formula of Example 1.

[Table 7]

| | TEER | Lucifer Yellow (%) | $P_{app} \times 10^{-6}$ cm/s) | |
|---|---|---|---|---|
| | | | at 0.5h | at 4h |
| Control (Transport Buffer (A)) | 5075 | 0.02% | 0.3 | 0.4 |
| DHA-DODAP | 6340 | 0.02% | 0.4 | 0.1 |
| EPA-DODAP | 6030 | 0.01% | 0.8 | 0.2 |
| Arachidonic acid-DODAP | 5760 | 0.01% | 1.5 | 0.3 |
| Isostearic acid*-DODMA | 5850 | 0.01% | 34.4 | 5.4 |
| Isostearic acid**-DODAP | 5750 | 0.01% | 5.0 | 1.6 |
| Isostearic acid**-DODMA | 5360 | 0.01% | 6.8 | 1.3 |
| Linoleic acid-DODAP | 5920 | 0.10% | 0.2 | 0.1 |
| Linolenic acid-DODAP | 6240 | 0.01% | 0.6 | 0.2 |
| Lactic acid-DODMA | 6160 | 0.02% | 0.9 | 0.2 |
| Lactic acid-DOTMA | 850 | 0.02% | 1.1 | 0.5 |
| Standard value | > 1000 Ω | < 1% | | |
| *: Isostearic acid (branched-chain), **: Isostearic acid (straight chain) | | | | |

The results showed that all of the ionic liquids had higher permeation coefficient and enhanced the permeability of

the drug as compared to the control, in particular, the combinations of isostearic acid and each cationic lipid produced remarkable permeation-enhancing effects.

Example 3: Evaluation of permeability of drug by Parallel Artificial Membrane Permeation Assay (PAMPA) (1)

The permeability of a drug with low solubility and/or low permeability in each type of ionic liquid preparation was evaluated by the PAMPA. In this assay, VZ185 and BI-3663 were used as a drug with low solubility and/or low permeability, and isostearic acid-DODMA, isostearic acid-DODAP and oleic acid-DODMA were used as an ionic liquid. As the control, PBS was used.

Specifically, the permeability of the drugs was evaluated by Parallel Artificial Membrane Permeability Assay-Skin Kit (BioAssay Systems) according to the following procedures.

(1) Preparation of phosphate buffer solution (PBS)
One tablet of Phosphate Buffered Saline Tablet (Sigma) was dissolved in 200 mL of water to prepare PBS.
(2) Preparation of sample solution

VZ185 (1.5 mg) was mixed with 1% TWEEN 80 (1.5 mL) to prepare 1 mg/mL of VZ185 solution. Each organic acid (0.025 mmol) which is an anion ingredient and each cationic lipid (0.025 mmol) which is a cation ingredient were mixed with PBS (4.98 mL) prepared in the above (1) to prepare an ionic liquid composition in which each ingredient is contained in an amount of 5 mM. 1 mg/mL of VZ185 solution (25 μL) and the ionic liquid composition (475 μL) were mixed to prepare 50 μg/mL of a drug-ionic liquid composition. Also, PBS (475 μL) was mixed with 1 mg/mL of VZ185 solution (25 μL) to prepare 50 μg/mL of a drug-control solution as the control.

4% Lecithin/dodecane solution (5 μL) was then added into each donor well and allowed to stand. PBS (300 μL) was added into each acceptor well and 50 μg/mL of the drug-ionic liquid composition (200 μL) was added into each donor well, and the mixture was incubated in a thermostatic bath at 37°C. After 30, 240 and 1440 minutes of the incubation, 300 μL of sample was collected from each acceptor well and the collected sample was used as sample solution. PBS (300 μL) was added to each acceptor well after the sample was collected, and then the mixture was incubated in a thermostatic bath at 37°C again. The sample solution of BI-3663 was prepared according to the same procedure.

Also, control solution was prepared according to the same method except that a drug-control solution was used instead of the drug-ionic liquid composition.

(3) Assay Method

Donepezil hydrochloride (10 mg) was dissolved in methanol (100 mL) to prepare 100 μg/mL of IS solution. 100 μg/mL of the IS solution (1 mL) was accurately taken, methanol was added thereto and adjusted to exactly 100 mL, and the solution was used as IS standard undiluted solution. The IS standard undiluted solution (2 mL) was accurately taken, acetonitrile was added thereto and adjusted to exactly 200 mL, and the solution was used as IS-acetonitrile solution (10 ng/mL donepezil/acetonitrile solution).

The sample solution or control solution (100 μL) and the IS-acetonitrile solution (400 μL) were mixed, the mixture was centrifuged at 4°C, 3,000 x g for 10 minutes, and then the concentration of the drug in the supernatant was measured by LC-MS/MS under the condition in the table below.

[Table 8]

| | |
|---|---|
| HPLC system | LC-2040C (Shimadzu Corporation) |
| Mass spectrometer | LCMS8050 (Shimadzu Corporation) |
| Column | ACQUITYUPLC CSH C18 (2.1 x 50 mm I.D., 1.7 μm) |
| Column temperature | 40°C |
| Injection amount | 5 μL |
| Flow rate | 0.5 mL/min |
| Analysis time | 12 minutes |
| Analysis time | A solution: 0.1% aqueous formic acid solution<br>B solution: 0.1% formic acid/acetonitrile |

[Table 9]

| Time program | | |
|---|---|---|
| Time (min) | A solution (%) | B solution (%) |
| 0 | 97 | 3 |
| 1 | 97 | 3 |
| 3.5 | 70 | 30 |
| 3.6 | 10 | 90 |
| 6.5 | 10 | 90 |
| 6.6 | 97 | 3 |

[Table 10]

| Setting condition of Multiple Reaction Monitoring (MRM) | | |
|---|---|---|
| Drug | Precursor ion | Product ion |
| VZ185 | 996.15 | 309.15 |
| BI-3663 | 919.00 | 442.95 |

The results are shown in Fig. 1 and Fig. 2. The results showed that the absorbability of VZ185 and BI-3663 was remarkably improved by the use of the ionic liquids.

Example 4: Evaluation of permeability of drug by Parallel Artificial Membrane Permeation Assay (PAMPA) (2)

The permeability of a drug with low solubility and/or low permeability in each type of ionic liquid preparation was evaluated by the PAMPA. In this assay, bifonazole was used as a drug with low solubility and/or low permeability and isostearic acid-DODMA, isostearic acid-DODAP and oleic acid-DODMA were used as an ionic liquid. As a control, PBS was used.

Specifically, the permeability of bifonazole was evaluated by Parallel Artificial Membrane Permeability Assay-Skin Kit (BioAssay Systems) according to the same procedure as that of Example 3.

The results are shown in Fig. 3. The results showed that the absorbability of bifonazole was remarkably improved by the use of the ionic liquid.

Example 5: Evaluation of permeability of drug by Caco-2 cell membrane permeation assay (3)

The permeability of a drug with low solubility and/or low permeability in each type of ionic liquid preparation was evaluated by the Caco-2 cell membrane permeation assay. In this assay, VZ185 was used as a drug with low solubility and/or low permeability and oleic acid-DODMA and oleic acid-DODAP were used as an ionic liquid. As the control, Transport Buffer (A) was used.

Specifically, the permeability of VZ185 was evaluated according to the procedure of Example 1.

The results are shown in Fig. 4. The results showed that oleic acid-DODMA and oleic acid-DODAP produced remarkable effects of enhancing the gastrointestinal absorption of the drug and that the absorbability of VZ185 was remarkably improved by the use of the ionic liquids.

Example 6: Evaluation of permeability of drug by Caco-2 cell membrane permeation assay (4)

The permeability of a drug with low solubility and/or low permeability in each type of ionic liquid preparation was evaluated by the Caco-2 cell membrane permeation assay. In this assay, BI-3663 was used as a drug with low solubility and/or low permeability and isostearic acid-DODMA, oleic acid-DODMA and oleic acid-DODAP were used as an ionic liquid. As the control, Transport Buffer (A) was used.

Specifically, the permeability of BI-3663 was evaluated according to the procedure of Example 1.

The results are shown in Fig. 5. The results showed that isostearic acid-DODMA, oleic acid-DODMA and oleic acid-DODAP produced remarkable effects of enhancing the gastrointestinal absorption of the drug and that the absorbability of BI-3663 was remarkably improved by the use of the ionic liquids.

Example 7: Evaluation of permeability of drug by Caco-2 cell membrane permeation assay (5)

The permeability of a drug with low solubility and/or low permeability in each type of ionic liquid preparation was evaluated by the Caco-2 cell membrane permeation assay. In this assay, bifonazole was used as a drug with low solubility and/or low permeability and oleic acid-DODMA was used as an ionic liquid. As the control, Transport Buffer (A) was used.
Specifically, the permeability of bifonazole was evaluated according to the procedure of Example 1.
The results are shown in Fig. 6. The results showed that oleic acid-DODMA produced remarkable effects of enhancing the gastrointestinal absorption of the drug and that the absorbability of bifonazole was remarkably improved by the use of the ionic liquid.

Example 8: Evaluation of permeability of drug by Caco-2 cell membrane permeation assay (6)

The permeability of a drug with low solubility and/or low permeability in each type of ionic liquid preparation was evaluated by the Caco-2 cell membrane permeation assay. In this assay, ritonavir (drug with low solubility which is almost insoluble in water) was used as a drug with low solubility and/or low permeability and isostearic acid-DODMA was used as an ionic liquid. As the control, Transport Buffer (A) was used.
Specifically, the permeability of ritonavir was evaluated according to the procedure of Example 1.
The results are shown in Fig. 7. The results showed that isostearic acid-DODMA produced remarkable effects of enhancing the gastrointestinal absorption of the drug and that the absorbability of ritonavir was remarkably improved by the use of the ionic liquid.

Example 9: Evaluation of permeability of drug by Caco-2 cell membrane permeation assay (7)

The permeability of a drug with low solubility and/or low permeability in each type of ionic liquid preparation was evaluated by the Caco-2 cell membrane permeation assay. In this assay, furosemide (drug with low solubility which is almost insoluble in water) was used as a drug with low solubility and/or low permeability and isostearic acid-DODAP and oleic acid-DODAP were used as an ionic liquid. As the control, Transport Buffer (A) was used.
Specifically, the permeability of furosemide was evaluated according to the procedure of Example 1.
The results are shown in Fig. 8. The results showed that isostearic acid-DODAP and oleic acid-DODAP produced remarkable effects of enhancing the gastrointestinal absorption of the drug and that the absorbability of furosemide was remarkably improved by the use of the ionic liquids.
The above assay results showed that the absorbability of drugs with low solubility and/or low permeability was remarkably improved by the use of ionic liquids.

INDUSTRIAL APPLICABILITY

According to the present invention, the absorbability of even a drug with low solubility and/or low permeability, which is difficult to be applied as an oral preparation, are remarkably improved, and thus the drug can be prepared as an oral preparation. In addition, a transmucosal absorption enhancer or a gastrointestinal absorption enhancer comprising the ionic liquid of the present invention can enhance the transmucosal or gastrointestinal absorption of a drug with low solubility and/or low permeability.

## Claims

1. A pharmaceutical composition comprising a drug with low solubility and/or low permeability and an ionic liquid, wherein the ionic liquid comprises an anion ingredient and a cation ingredient, the anion ingredient is oleic acid or isostearic acid, and the cation ingredient is selected from a cationic lipid.

2. The pharmaceutical composition according to claim 1, wherein the cationic lipid is DODAP, DODMA or DOTMA.

3. The pharmaceutical composition according to claim 1 or 2, wherein the anion ingredient is oleic acid and the cation ingredient is DODAP or DODMA.

4. The pharmaceutical composition according to any one of claims 1 to 3, wherein the drug with low solubility and/or low permeability is PROTAC.

5. An oral preparation comprising the pharmaceutical composition according to any one of claims 1 to 4.

**6.** A transmucosal absorption enhancer comprising an ionic liquid, wherein the ionic liquid comprises an anion ingredient and a cation ingredient, the anion ingredient is oleic acid or isostearic acid, and the cation ingredient is selected from a cationic lipid, provided that a drug is not comprised.

**7.** A preparation comprising a drug with low solubility and/or low permeability and the transmucosal absorption enhancer according to claim 6.

**8.** A gastrointestinal absorption enhancer comprising an ionic liquid, wherein the ionic liquid comprises an anion ingredient and a cation ingredient, the anion ingredient is oleic acid or isostearic acid, and the cation ingredient is selected from a cationic lipid, provided that a drug is not comprised.

**9.** The gastrointestinal absorption enhancer according to claim 8, wherein the cationic lipid is DODAP, DODMA or DOTMA.

**10.** The gastrointestinal absorption enhancer according to claim 8 or 9, wherein the anion ingredient is oleic acid and the cation ingredient is DODAP or DODMA.

**11.** An oral preparation comprising a drug with low solubility and/or low permeability and the gastrointestinal absorption enhancer according to any one of claims 8 to 10.

**12.** The oral preparation according to claim 11, wherein the drug with low solubility and/or low permeability is PROTAC.

[Fig. 1]

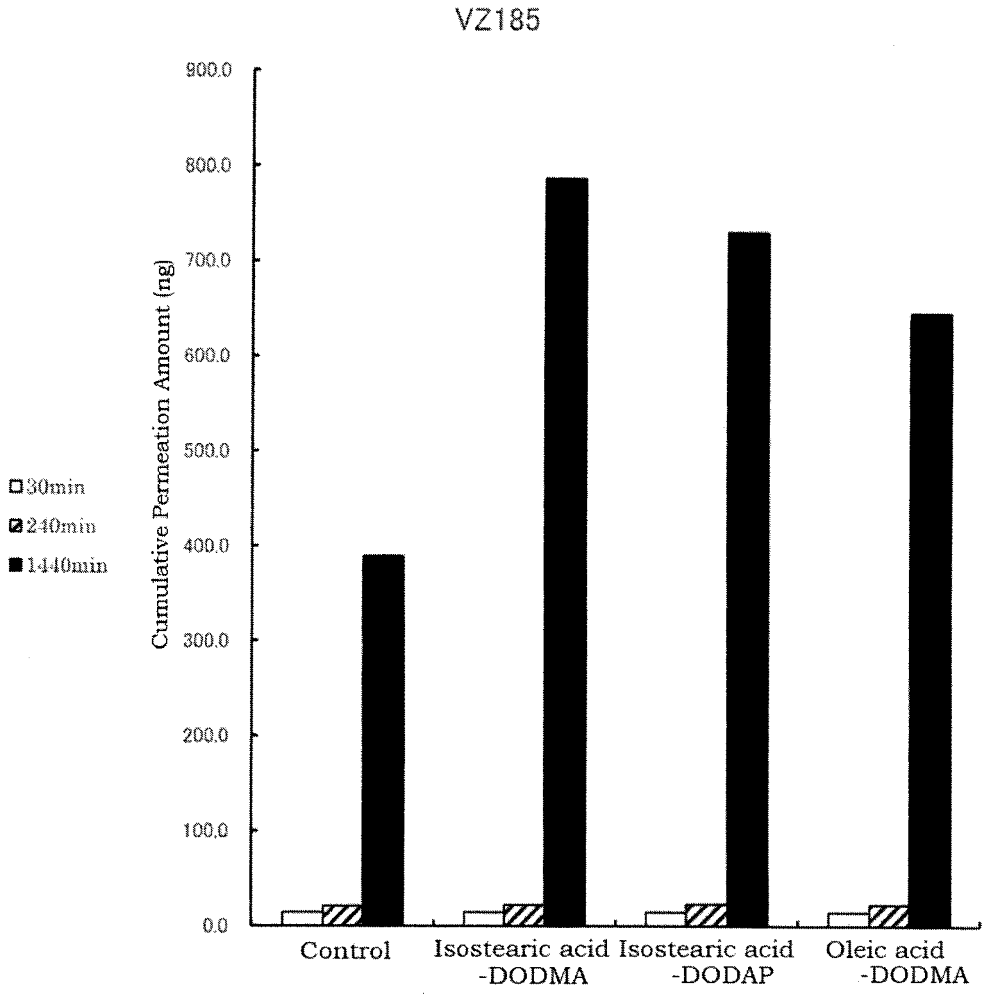
VZ185
Cumulative Permeation Amount (ng)
900.0
800.0
700.0
600.0
500.0
400.0
300.0
200.0
100.0
0.0
30min
240min
1440min
Control
Isostearic acid -DODMA
Isostearic acid -DODAP
Oleic acid -DODMA

[Fig. 2]

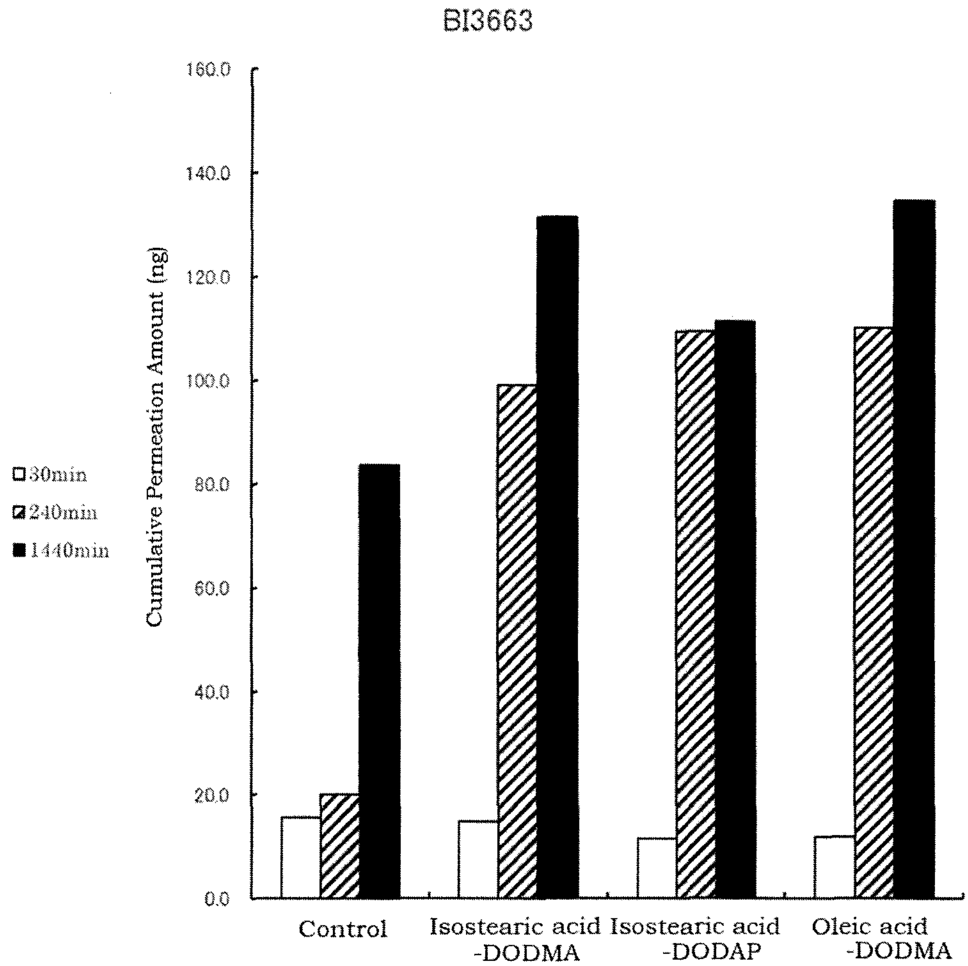
BI3663
Cumulative Permeation Amount (ng)
160.0
140.0
120.0
100.0
80.0
60.0
40.0
20.0
0.0
30min
240min
1440min
Control
Isostearic acid -DODMA
Isostearic acid -DODAP
Oleic acid -DODMA

[Fig. 3]

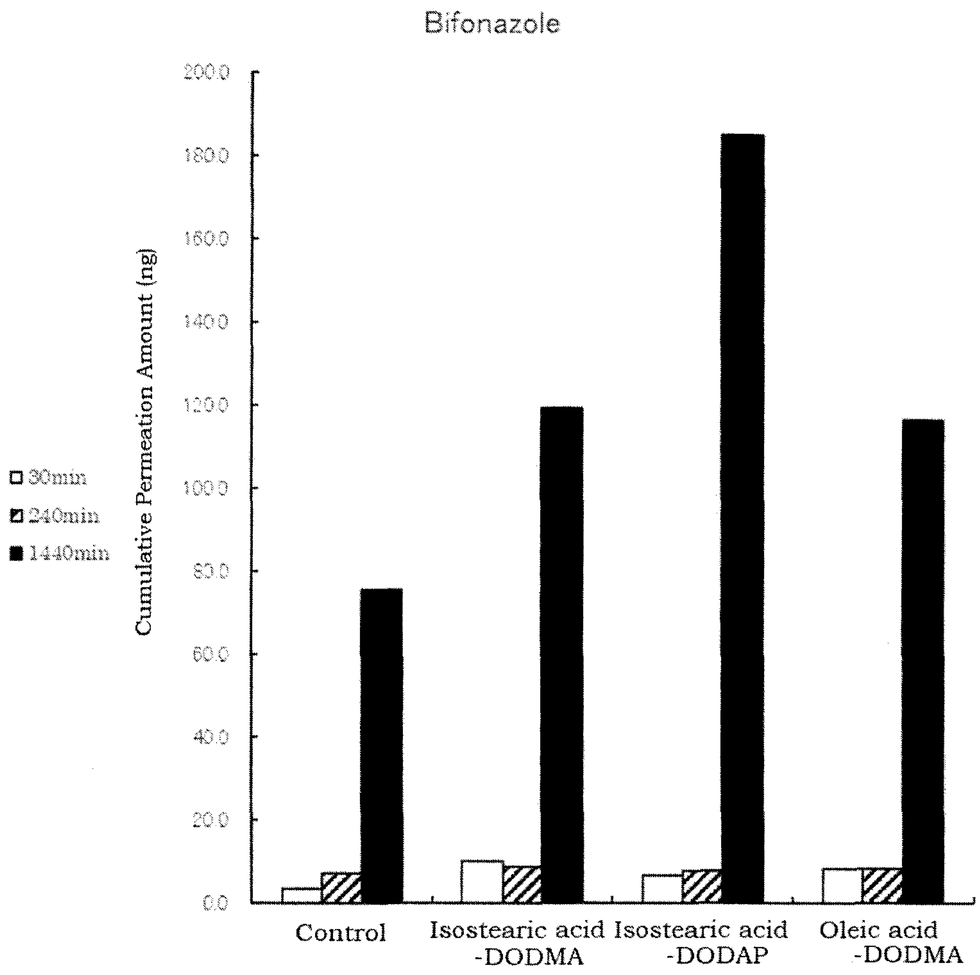
Bifonazole
Cumulative Permeation Amount (ng)
200.0
180.0
160.0
140.0
120.0
100.0
80.0
60.0
40.0
20.0
0.0
30min
240min
1440min
Control
Isostearic acid -DODMA
Isostearic acid -DODAP
Oleic acid -DODMA

[Fig. 4]

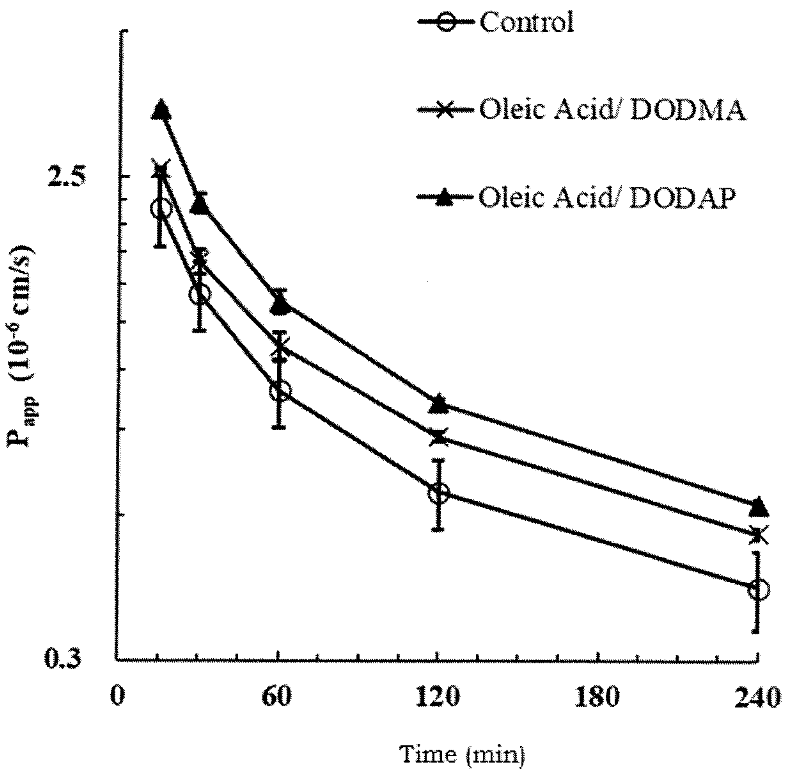
Control
Oleic Acid/ DODMA
Oleic Acid/ DODAP
2.5
0.3
$P_{app}$ ($10^{-6}$ cm/s)
0
60
120
180
240
Time (min)

[Fig. 5]

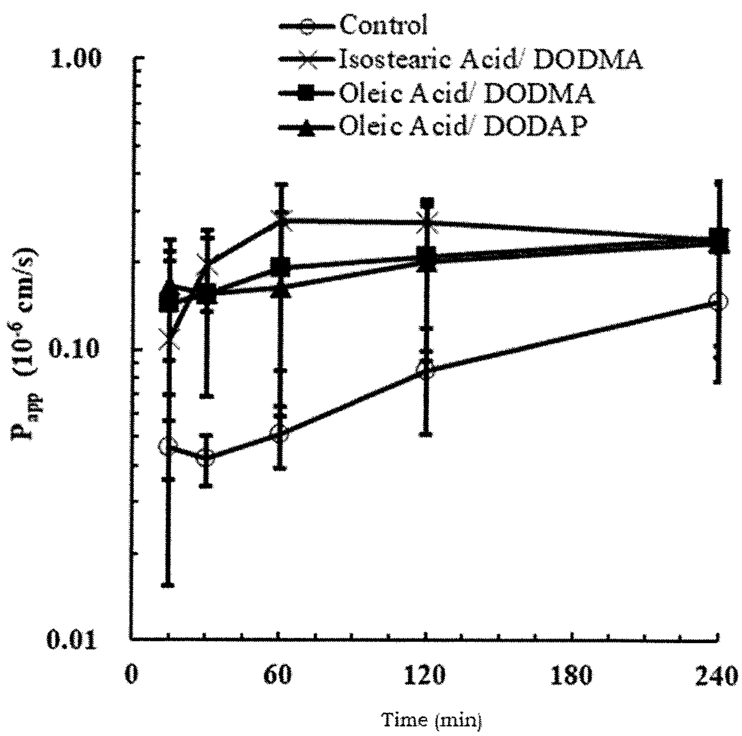
Control
Isostearic Acid/ DODMA
Oleic Acid/ DODMA
Oleic Acid/ DODAP
1.00
0.10
0.01
$P_{app}$ ($10^{-6}$ cm/s)
0
60
120
180
240
Time (min)

[Fig. 6]

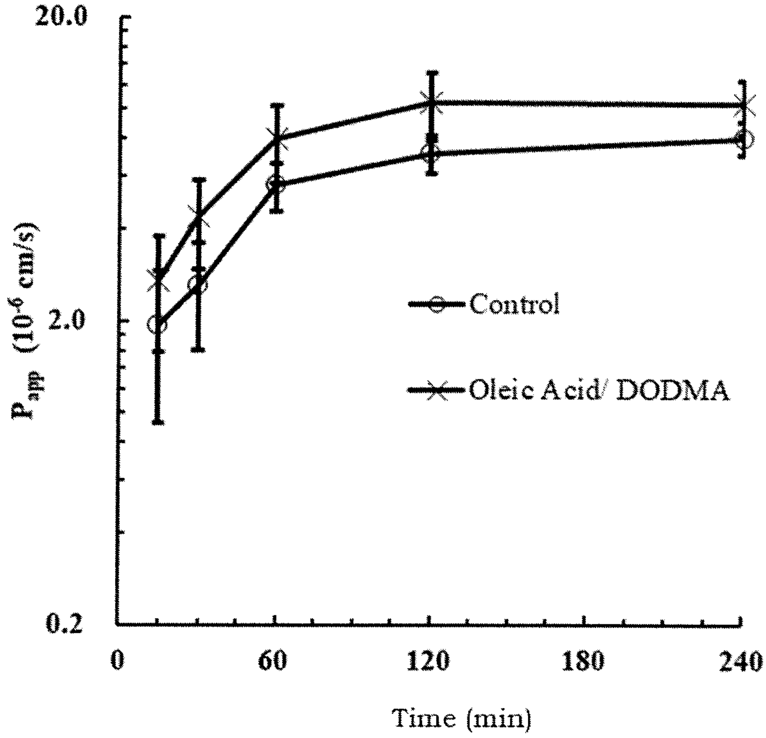
20.0
2.0
0.2
$P_{app}$ ($10^{-6}$ cm/s)
0
60
120
180
240
Time (min)
Control
Oleic Acid/ DODMA

[Fig. 7]

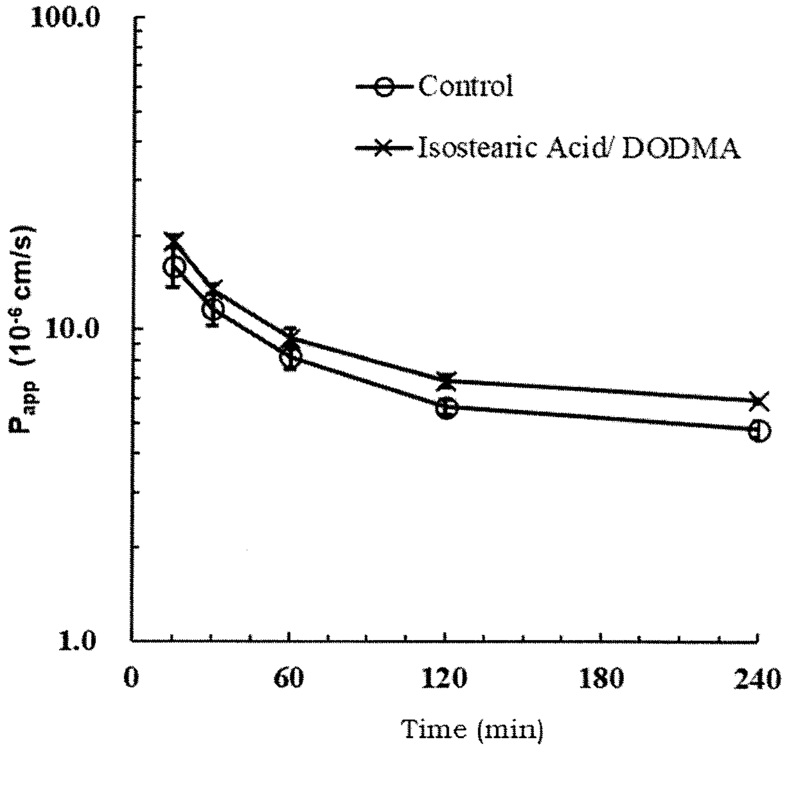
100.0
10.0
1.0
$P_{app}$ ($10^{-6}$ cm/s)
0
60
120
180
240
Time (min)
Control
Isostearic Acid/ DODMA

[Fig. 8]

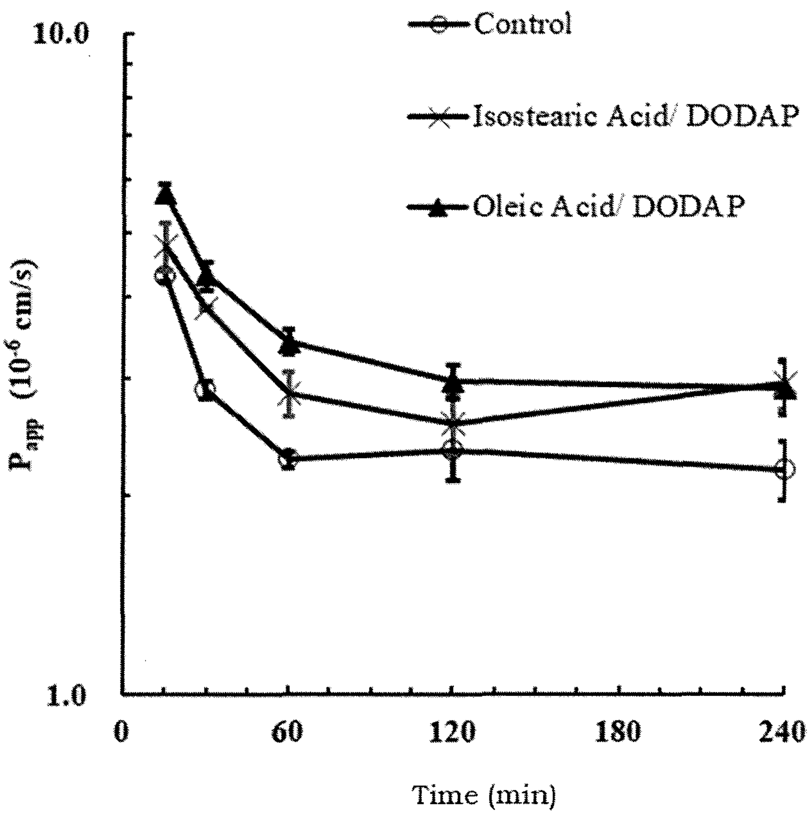
Control
Isostearic Acid/ DODAP
Oleic Acid/ DODAP
10.0
1.0
$P_{app}$ ($10^{-6}$ cm/s)
0
60
120
180
240
Time (min)

## INTERNATIONAL SEARCH REPORT

International application No. **PCT/JP2023/013657**

**A. CLASSIFICATION OF SUBJECT MATTER**

***A61K 47/12***(2006.01)i; ***A61K 9/08***(2006.01)i; ***A61K 47/18***(2017.01)i
FI: A61K47/12; A61K47/18; A61K9/08

According to International Patent Classification (IPC) or to both national classification and IPC

**B. FIELDS SEARCHED**

Minimum documentation searched (classification system followed by classification symbols)

A61K47/12; A61K9/08; A61K47/18

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY/MEDLINE/EMBASE/BIOSIS (STN); JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | WO 2021/192861 A1 (KYUSHU UNIV.) 30 September 2021 (2021-09-30) paragraphs [0053]-[0054], [0057] | 1 |
| Y | | 4 |
| A | | 2-3, 5-12 |
| Y | JP 2018-531983 A (YALE UNIV.) 01 November 2018 (2018-11-01) claims 1-18, 24, paragraph [0025] | 4 |
| A | JP 2021-503468 A (PRES. AND FELL. OF HARVARD COLLEGE) 12 February 2021 (2021-02-12) | 1-12 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 June 2023** | **20 June 2023** |
| Name and mailing address of the ISA/JP<br>**Japan Patent Office (ISA/JP)<br>3-4-3 Kasumigaseki, Chiyoda-ku, Tokyo 100-8915<br>Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2023/013657**

| Patent document cited in search report | Publication date (day/month/year) | Patent family member(s) | Publication date (day/month/year) |
|---|---|---|---|
| WO 2021/192861 A1 | 30 September 2021 | EP 4130013 A1<br>paragraphs [0050], [0054]<br>CN 115298190 A<br>KR 10-2022-0159986 A | |
| JP 2018-531983 A | 01 November 2018 | US 2017/0121321 A1<br>claims 1-18, 24, paragraph [0049]<br>WO 2017/079267 A1<br>EP 3370715 A1<br>CN 108366992 A<br>KR 10-2018-0097530 A | |
| JP 2021-503468 A | 12 February 2021 | US 2020/0289421 A1<br>WO 2019/099837 A1<br>EP 3709974 A1<br>CN 111918644 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2020073533 A
- US 10278923 B


### Non-patent literature cited in the description


- A theoretical basis for a biopharmaceutic drug classification: the correlation of in vitro drug product dissolution and in vivo bioavailability. *Pharm Res.*, March 1995, vol. 12 (3), 413-20